# EUROPÄISCHE PATENTANMELDUNG

(11) **EP 2 786 800 A1**
(43) Veröffentlichungstag der Anmeldung: **08.10.2014**
(21) Anmeldenummer: 14001120.6
(22) Anmeldetag: 26.03.2014
(51) Int. Cl.: B01J 19/08, C02F 1/46, C02F 1/467, C02F 1/461

(54) **Ionenerzeuger für die Entkeimung (Behandlung) von Wasser u. Luft mit ionisiertem Sauerstoff**

(30) Priorität: 02.04.2013 DE 102013005606
(71) Anmelder: Emmerich, Steffen, 06886 Lutherstadt Wittenberg (DE)
(72) Erfinder: Emmerich, Steffen, 06886 Lutherstadt Wittenberg (DE)

(57) **Zusammenfassung**

2.1
Ionenerzeuger zum Erzeugen von Sauerstoffionen, für die Behandlung sowie Entkeimung von Wasser und Luft, auf Basis der natürlichen Oxidation von Sauerstoff. Die Anwendungsgebiete sind Luft- und Wasseraufbereitung, wie z.B. Geruchsneutralisation, Entkeimung oder Schadstoffabbau.

Der Ionenerzeuger besteht aus einer porösen, luftdurchlässigen Anode (1) und aus einer Kathode (3), die sich im inneren eines doppelwandigen Isolators (4) befindet. Der Isolator (4) wird im inneren der zylindrischen Anode (1) befestigt und er ist mit Löchern vorsehen. Durch Zuführung von Luft (9) in den Innenraum des doppelwandigen Isolators (4), kann diese durch die im Isolator (4) befindlichen Löcher hindurch zu der porösen Anode (1) strömen. Beim durchströmen der Luft durch die porösen Anode (1), werden die in der Luftbefindlichen Sauerstoffanteile ionisiert und die natürliche Oxidation mit Keimen beginnt unmittelbar bzw. während der Ionisation.

## Beschreibung

Es ist bekannt, dass durch anlegen von Hochspannung an zwei gegenüberliegenden Elektroden, welche durch eine Glasröhre voneinander getrennt sind (Ionisationsröhre), Koronaentladungen entstehen und Elektronenemission an die Luft abgegeben werden (z.B. BENTAX-Röhre; Fa. BENTAX Umwelt Technik GmbH).

Weiter ist bekannt, dass sich im inneren dieser geschlossen Glasröhre die Anode und an der Außenseite der Glasröhre die Katode befindet. Die zu ionierende Luft fließt an der Katode vorbei und der in der Luft befindliche Sauerstoff wird ionisiert. Jedoch steht das hohe Oxidationspotential der Ionen nur für eine befristete Zeit bzw. Strecke, welche max. ein Meter nach ihrer Entstehung ausmacht, zur Verfügung.

Durch ionische Bindung erfolgt die Bildung von Sauerstoffradikalen in Form von kleinen Clustern. Diese Sauerstoffradikale dienen der Luft-u. Wasserdesinfektion sowie der Luft-u. Wasseraufbereitung, wie z.B. Geruchsneutralisation, Entkeimung oder Schadstoffabbau. (Wikipedia, Artikel "Ionisationsröhre").

Der im Patentanspruch 1 angegebenen Erfindung liegt zugrunde, dass durch das Ionisationsverfahren bekannter Ionisationsröhren, nur ein Bruchteil der zu ionisierenden Luft erreicht wird, da diese nur dann ionisiert wird, wenn sie an der Ionisationsröhre vorbei streift und mit der Katode in Kontakt kommt. Des Weiteren kann eine Erhöhung der Ionisationsleistung und damit der Wirkungsgrad, nur durch Änderung von Anzahl bzw. Bauart der Röhren erreicht werden.

Dieses Problem wird durch die folgenden, im Patentanspruch 1 aufgeführten Merkmale, gelöst:
- Einbau des Ionenerzeugers in das zu behandelnde Medium (Behandlung/Entkeimung von Wasser)
- Direkte Durchströmung des Ionenerzeugers mit der zu behandelnden Luft (Behandlung/Entkeimung von Luft)
- Dadurch Erzeugung von Ionen genau dort wo sie benötigt werden, eine Reaktion mit Keimen und Schadstoffen findet unmittelbar nach bzw. während der Entstehung von Sauerstoffradikalen statt
- Durch den einfachen Aufbau ist der Ionenerzeuger für Wartungs-u. Reparaturarbeiten, leicht zerleg- bzw. ein- u. ausbaubar
- Ein weiterer Vorteil der für den einfachen Aufbau spricht, sind die niedrigen Produktionskosten, welche in Verbindung mit der Steigerung von Leistungsfähigkeit und Optimierung des Wirkungsgrad stehen

Ein weiterer Vorteil der Erfindung ist im Patentanspruch 2 gegeben, welche durch die folgenden Funktionen und Komponenten erreicht wird.
- Eine Leistungsregelung und damit verbundene Optimierung des Wirkungsgrades kann durch Veränderung der Frequenz, Spannung und Frequenztaktung, mit dem Einsatz von Frequenzumrichtern und einen speziell an den Ionisator angepassten Hochspannungswandler erfolgen.

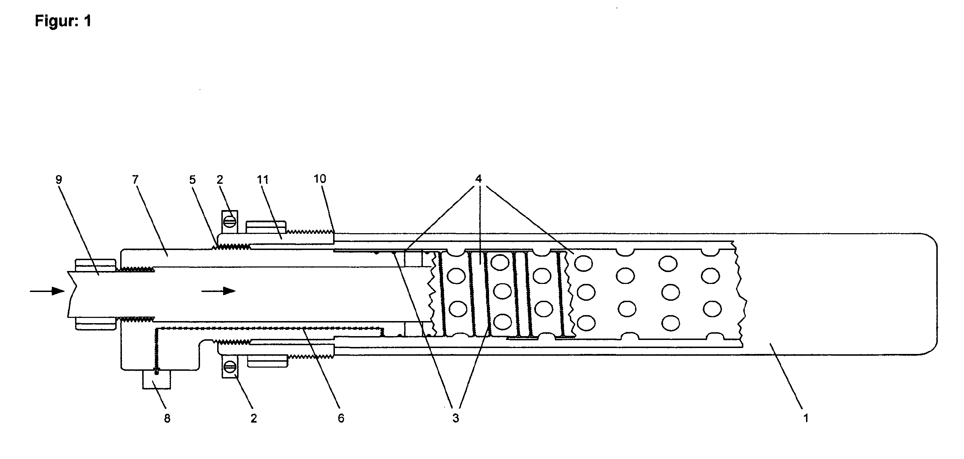

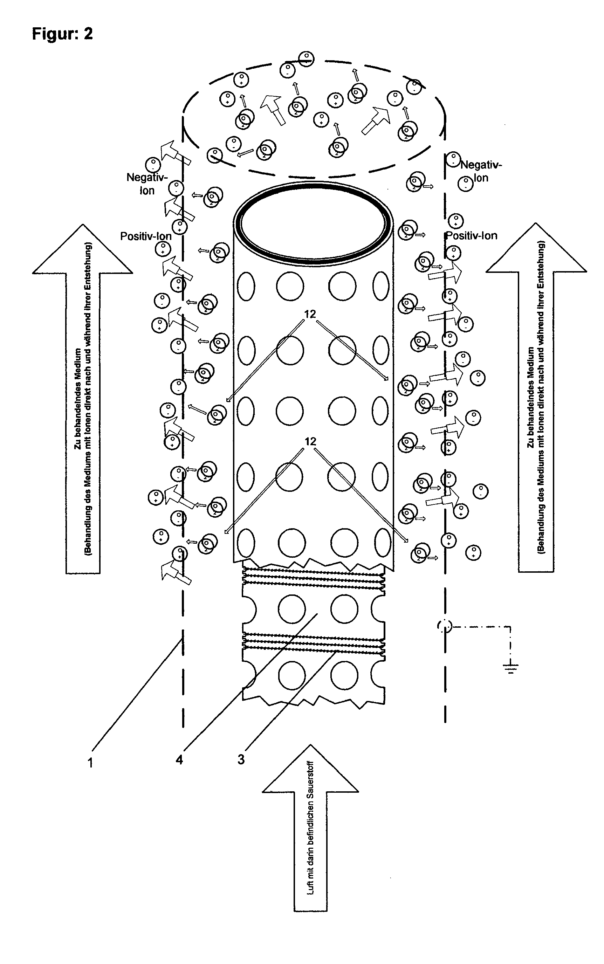

Der Aufbau des Ionenerzeugers an einem Ausführungsbeispiel wird in der Zeichnung Figur 1 sowie das Funktionsprinzip in Figur 2 dargestellt und im folgenden näher erläutert.
Die Ausführungsarten (eckig, rund, etc.) des Ionenerzeugers können je nach Einsatzbedingung unterschiedlich erfolgen. (gewählte Ausführungsart in den Darstellungen ist rund)

### Figur 1 - Aufbau des Ionisators

Der lonisator (Figur 1) besteht aus der Anode (1), welche aus einem prösen, luftdurchlässigen und elektrostatische Felder leitendendem Material besteht. Über eine Schweißverbindung (10) ist die Anode (1) mit einer Ededelstahl-Gewindemuffe (11) untrennbar verbunden. Die Anode wird mit zwei Potentialausgleichsleitungen über die Erdanschlußkontakte (2), an der Ededelstahl-Gewindemuffe (11), mit dem Erdpotential verbunden und somit geerdet.

Die Katode (3), ebenfalls aus einem elektrostatische Felder leitendendem Material (Ausführung als Band, Platte oder Geflecht, etc.), befindet ich im inneren eines doppelwandigen Isolators (4), welcher mit Löchern versehen ist und aus einem gegen Hochspannung und Strom isolierenden Material besteht.

Der Isolator (4) mit der Innenliegenden Katode (3), wird im inneren der Anode (1) befestigt (Verschraubung etc.), somit sind die beiden Elektroden durch eine lösbare Verbindung (5) fest miteinander verbunden.

Durch eine leitende Verbindung (6) (Draht, Kabel, o.ä.), welche sich im inneren des Anschlusssockels (7) von dem doppelwandigen Isolator (4) befindet, wird die Katode (3) mit dem elektrischen Hochspannungsanschluss eines Hochspannungssteckkontaktes (8) am Anschlusssockel (7) verbunden.

Des Weiteren befindet sich am Anschlusssockel (7) des doppelwandigen Isolators (4), die Einführung (Verschraubung o.ä.) für den Lufteinlass(9) in den Isolator (4) mit der Katode (3).

Durch die Verwendung einer flinken Hochspannungsfeinsicherung in der Spannungsversorgung, für die Katode sowie eine redundante Erdung der Anode, ist gewährleistet das im Fehlerfall (Isolationsschaden am Isolator; Kurzschluss, Erdschluss, etc.) eine sichere Abschaltung des Ionenerzeugers von der Spannungsversorgung erfolgt. Des Weiteren wird durch den Frequenzumrichter die permanente Stromaufnahme gemessen und bei Abweichung des parametrierten Grenzwertes eine Störung signalisiert, bzw. es erfolgt die Abschaltung des Ionenerzeugers.

### Figur 2 - Funktionsprinzip des Ionenerzeugers

Durch anlegen einer in Frequenz und Frequenztaktung modulierten Hochspannung, wird zwischen bzw. in der Anode (1) und doppelwandigen Isolator (4) mit innenliegender Katode (3) (Isolator liegt direkt an der Innenseite der Anode (1) an), ein elektrostatisches Feld (12) erzeugt.

Durch Zuführung von Luft, mit darin befindlichen Sauerstoff, in den Innenraum des doppelwandigen Isolators (4), kann diese durch die im Isolator (4) befindlichen Löcher hindurch zu der porösen Anode (1) strömen.

Das elektrostatische Hochspannungsfeld (12) erzeugt an sowie im inneren der Anode (1) Koronaentladungen. Beim durchströmen der Luft durch die poröse Anode (1), werden die in der Luftbefindlichen Sauerstoffanteile ionisiert und die natürlich Oxidation mit Keimen beginnt unmittelbar bzw. während der Ionisation.

### Verfahren zum behandeln von Wasser

Bei der Behandlung von Wasser, strömt die ionisierte Luft durch die Anode (1) direkt in das zu behandelnde Wasser ein und die Sauerstoffradikale regieren mit den im Wasser befindlichen Keimen.

### Verfahren zum behandeln von Luft

Bei der Behandlung von Luft wir diese bereits beim durchströmen der Anode durch die Reaktion der entstehenden Sauerstoffradikale entkeimt.

### Vorteil

Der große Vorteil an dieser Erfindung ist, dass die gesamte, für die Behandlung zur Verfügung stehende Luft, durch den Ionisator stömen muss. Prinzipiell wird der gesamte in der Luft befindliche Sauerstoffanteil von der porösen und sehr feinporigen Membran der Anode ionisiert.

Der Wirkungsgrad hat sich somit nahezu auf 100% erhöht, wobei der Energie- u. der Wartungsaufwand erheblich gesunken ist, da nur noch ein Ionenerzeuger, satt mehrerer Ionisationsröhren, eingesetzt werden muss.

### Bezugszeichenliste zu Figur 1

- 1: Poröses Elektrostatische-Felder leitender Werkstoff (Anode) - Ausströmung des Ionisierten Sauerstoffanteils der einströmenden Luft
- 2: Anschluss Anode (Redundante Festverbindung an Erdpotential mittels Erdungskabel)
- 3: Katode (aus Spannung- u. Stromleitenden Werkstoff in Form und Art anpassbar)
- 4: Doppelwandiger Isolator mit innenliegender Katode und Löchern für Luftdurchsatz zur Anode (Werkstoff isolierend gegenüber Spannung und Strom)
- 5: Lösbare Verbindung zwischen Anode und Katode Strom
- 6: Leitende Verbindung zwischen Katode und Hochspannungssteckkontakt
- 7: Anschlusselement des Ionenerzeugers (Werkstoff isolierend gegenüber Spannung und Strom)
- 8: Anschluss Spannungsversorgung Katode (Anschluss mit Hochspannungskabel und Hochspannungsstecker IP64)
- 9: Lufteinlass in den Isolator mit der Katode
- 10: Untrennbare Schweißverbindung
- 11: Edelstahl-Gewindemuffe

## Patentansprüche

1. Ionenerzeuger zum Erzeugen von Sauerstoffionen, für die Behandlung sowie Entkeimung von Wasser und Luft, auf Basis der natürlichen Oxidation von Sauerstoff,
**dadurch gekennzeichnet,**
**dass** der Ionenerzeuger direkt in das zu behandelnde Medium eingebaut (Behandlung von Wasser) bzw. von diesem durchströmt wird (Behandlung von Luft).
Der Ionenerzeuger zeichnet sich durch seinen einfachen Aufbau aus, ist somit für Wartungsmaßnahmen leicht zu zerlegen und für Reparaturen schnell aus- bzw. einzubauen.
Durch den einfachen Aufbau werden Produktions- und Wartungskosten gesenkt, welche in Verbindung mit der Steigerung sowie Optimierung von Wirkungsgrad und Leistungsfähigkeit, für dieses Verfahren einhergehen.

2. Ionenerzeuger nach Patentanspruch 1,
**dadurch gekennzeichnet,**
**dass** die Produktion von Ionen und somit die Leistung des Gerätes, durch Anpassung von Frequenz, Spannung und Frequenztaktung, durch den Einsatz eines Frequenzumrichters inkl. eines für diesen Ionenerzeuger optimierten Hochspannungswandler, stufenlos regelbar ist. Eine Anpassung und Reaktion auf Situationsänderungen laufender Prozesse, für die der Ionenerzeuger eingesetzt wird, ist somit jederzeit möglich bzw. gegeben.
